# EUROPÄISCHE PATENTSCHRIFT

(11) **EP 0 649 022 B1**
(45) Veröffentlichungstag und Bekanntmachung des Hinweises auf die Patenterteilung: **04.11.1998**
(21) Anmeldenummer: 94890144.2
(22) Anmeldetag: 05.09.1994
(51) Int. Cl.: G01N 33/84, G01N 31/22, G01N 21/64

(54) **Optischer Indikator zur Bestimmung der Aktivität eines Ions in einer Probe**
Optical indicator for determining the activity of an ion in a sample
Indicateur optique pour la détermination de l'activité d'un ion dans un échantillon

(30) Priorität: 30.09.1993 AT 1970/93
(43) Veröffentlichungstag der Anmeldung: 19.04.1995
(73) Patentinhaber: AVL Medical Instruments AG, 8207 Schaffhausen (CH)
(72) Erfinder: Karpf, Hellfried, Dr., A-8043 Graz (AT)
(74) Vertreter: Krause, Walter, Dr. Dipl.-Ing.

(56) Entgegenhaltungen:
- EP-A- 0 207 392
- US-A- 5 096 832

## Beschreibung

Die Erfindung betrifft einen optischen Indikator zur Bestimmung der Aktivität eines Ions in einer Probe, welche den Indikator zumindest indirekt kontaktiert, wobei der Indikator einen Ionophor und zumindest zwei funktionelle Gruppen aufweist, die einen Elektronen-Donator-Akzeptor (EDA) Komplex bilden, dessen Konformation sich durch die Bindung des zu messenden Ions an den Ionophor unter Auflösung des EDA-Komplexes derart ändert, daß sich zumindest eine der optischen Eigenschaften des Indikators meßbar ändert.

Aus der Literatur ist eine Anzahl verschiedener Verfahren zur Bestimmung von geladenen, d. h. ionischen Probeninhaltsstoffen bekannt. Mit diesen Verfahren ist sowohl die Bestimmung von chemischen Elementen in ihrer ionischen Form als auch die Bestimmung von geladenen Molekülen möglich. Die häufigste in der Praxis angewandte Methode ist dabei die Bestimmung von chemischen Elementen in ihrer ionischen Form mit sogenannten ionen-sensitiven Elektroden (sogenannten ISE-Elektroden). Grundlage dieser Meßtechnik ist die Verwendung von ionenselektiven Molekülen, die in der Lage sind, an der Sensormembran hochspezifisch und selektiv bestimmte Ionen aufzunehmen, dabei ein Potential zu erzeugen, das in der Folge meßtechnisch aufgenommen, verstärkt und einer Meßwertanzeige zugeführt wird.

Die Bestimmung dieser geladenen chemischen Elemente mit Hilfe optischer Analysenmethoden unter Verwendung von Sensoren (sogenannten Optoden) erscheint hingegen bis zum heutigen Zeitpunkt schwieriger. Es gibt Verfahren, bei welchen beispielsweise beschrieben wird, daß ein Ionophor in eine Membran eingebettet wird, daß in die selbe Membran ein potentialsensitiver Fluorophor eingebettet wird, daß diese Membran an ihrer Grenzfläche mit einer Probe in Kontakt tritt, daß aus der Probe ein Elektrolyt von einem Ionencarrier erkannt und aufgenommen wird, daß sich dadurch das Potential an der Grenzfläche verändert, und daß in der Folge der potentialsensitive Fluorophor seine spektroskopischen Eigenschaften ändert. Diese spektroskopischen Eigenschaften werden durch geeignete optische Meßeinrichtungen abgefragt und einer Meßwertbestimmung zugeführt. Ein derartiges Verfahren wird beispielsweise in der AT-B 384 677 beschrieben.

Ein weiteres derartiges Verfahren ist aus der US-A 4 645 744 bekannt geworden. Danach liegt in einer Membran sowohl ein Ionophor als auch ein Chromophor vor. Das Wirkprinzip besteht darin, daß bei Aufnahme eines geladenen Ions durch den Ionophor zur Ladungskompensation ein Ion aus dem Chromophor freigesetzt wird. Dieses freigesetzte Ion wird in die Probe abgegeben. Typischerweise handelt es sich dabei um ein Proton. Der Chromophor ist im wesentlichen eine Indikatorsäure, die nach Abgabe eines Protons in die entsprechende Indikatorbase umgesetzt wird, und daher über geänderte spektroskopische Eigenschaften verfügt. Nachteil dieser Methode ist, daß sie nur mit Proben zu realisieren ist, deren pH-Wert extrem genau bekannt ist, bzw. im Idealfall konstant ist.

Die Fehler in der pH-Werteinstellung der Probe bzw. in der pH-Wertbestimmung der Probe gehen exponentiell in das Meßergebnis für die Bestimmung des geladenen chemischen Elementes ein, da pH = -log [H⁺].

Aus der US 5 096 832 A ist ein Verfahren zur Bestimmung von Kationen unter Verwendung substituierter Cyklohexane bekannt geworden. Es wird darin ein optischer Indikator auf der Basis eines Cyklohexans beschrieben, das mit zwei Ionophoren und zwei Chromophoren substituiert ist. Dabei sind in den Positionen 1 und 2 des Cyklohexans ionophorische Gruppen und in den Positionen 4 und 5 Chromophore substituiert. Ein Chromophor ist ein Elektronendonator, der andere ein Elektronenakzeptor. Die beiden Chromophore bilden einen Elektronen-Donator-Akzeptor-(EDA) Komplex. Durch die Bindung des zu messenden Ions ändert sich die Konformation des EDA-Komplexes derart, daß sich zumindest eine der optischen Eigenschaften des Indikators meßbar ändert.

Aufgabe der Erfindung ist es, optische Indikatoren vorzuschlagen, welche die Nachteile der bisher beschriebenen und bekannten Indikatoren bzw. der damit realisierten Meßverfahren vermeiden, welche universell für unterschiedliche Ionen anwendbar sind, wobei identische spektroskopische Parameter für unterschiedliche Ionen herrschen sollen. Diese Aufgabe wird erfindungsgemäß dadurch gelöst, daß die funktionellen Gruppen an den Ionophor gebunden sind und folgende Ionophore zur Bestimmung der Aktivität folgender Ionen Verwendung finden:

| ION | IONOPHOR |
|---|---|
| Li⁺ | N,N'-Diheptyl-N,N',5,5-tetramethyl-3,7-dioxanonandiamid |
| | |
| Na⁺ | N,N,N',N,-Tetracyclohexyl-1,2-phenylendioxydiacetamid |
| | |
| Ca⁺⁺ | (-)-(R,R)-N,N'-bis-(11-(ethoxycarbonyl)-undecyl)-N,N'-4,5-tetramethyl-3,6-dioxaoctan-diamid |
| | |
| Mg⁺⁺ | N,N',N"-tris-(3-(heptylmethylamino)-3-oxopropionyl)-8,8'-iminodioctylamin |

Ein EDA-Komplex besteht stets aus einem Donor-Molekül und einem Akzeptor-Molekül. Das Donor-Molekül kann beispielsweise ein einzelnes Elektron oder Elektronenpaar in ein pi-Orbital einer Doppelbindung oder eines aromatischen Systems (aromatischen Kohlenwasserstoffs) abgeben. Die Existenz eines EDA-Komplexes kann durch die Überprüfung des Elektronenspektrums des Komplexes festgestellt werden. EDA-Komplexe erzeugen typischerweise ein Spektrum (Charge-Transfer-Spektrum), das nicht der Summe der Einzelspektren der beiden Einzelmoleküle entspricht. Unter Konformation versteht man die räumliche Anordnung der Atome eines Moleküls oder Molekülverbandes.

Die Anordnung der beiden funktionellen Gruppen entlang des Skeletts des Molküls, welches zur Ionenerkennung (Ionophor) geeignet ist, ist dergestalt, daß bei Abwesenheit von Ionen eine freie Beweglichkeit des Skeletts die Ausbildung eines Charge-Transfer Komplexes zwischen den zumindest zwei funktionelle Gruppen zuläßt. Tritt ein Ion hinzu, so geht dieses mit dem Molekülskelett des Ionophors eine koordinative Bindung ein, die das Skelett neu orientiert, bzw. die Konformation derart ändert, daß der Charge-Transfer Komplex aufgebrochen wird. Spektroskopisch äußert sich dieser Vorgang entweder dadurch, daß sich die spektroskopischen Eigenschaften des Systems verändern, d.h. z.B. neue Absorptions- und/oder Fluoreszenzbanden beobachtet werden können; Absorptions- und/oder Fluoreszenzbanden verschwinden; die Abklingzeit sich deutlich ändert oder sich andere spektroskopische Größen meßbar verändern (Anisotropie, Zirkulardichroismus, etc.).

Bei Einbettung derartiger Indikatormoleküle in eine hydrophobe Matrix ist darauf zu achten, daß die Wechselwirkung der funktionellen Gruppen klein ist, da es bekannt ist, daß in einer hydrophoben Matrix Molekülwechselwirkungen stark verstärkt werden. In einer hydrophilen Matrix können auch funktionelle Gruppen mit stärkeren Wechselwirkungen ausgewählt werden.

Eine erfindungsgemäße Ausführungsvariante sieht vor, daß beide funktionellen Gruppen des Ionophors aus einem aromatischen Kohlenwasserstoff mit jeweils zumindest einem Substituienten bestehen, wobei der zumindest eine Substituent einer funktionellen Gruppe ein Elektronen-Donator und der zumindest eine Substituent der anderen funktionellen Gruppe ein Elektronen-Akzeptor ist. Für die Stabilität dieser CT-Komplexe sind die Eigenschaften der jeweiligen Substituenten von besonderer Wichtigkeit. Die Fähigkeit Elektronen anzuziehen bzw. an das Elektronensystem der aromatischen Kohlenwasserstoffe abzugeben, ist durch die Hammett-Gleichung beschrieben. In dieser Gleichung scheint die Substituentenkonstante Sigma als Leitgröße auf, welche Aufschluß darüber gibt, wie stark Elektronen "ziehend" oder Elektronen "schiebend" der jeweilige Substituent ist.

Eine weitere Ausführungsvariante sieht vor, daß eine funktionelle Gruppe des Ionophors einen aromatischen Kohlenwasserstoff aufweist, dessen zumindest ein Substituent ein Elektronen-Donator ist, sowie daß die andere funktionelle Gruppe des Ionophors ein Atom aus der Gruppe Chlor, Brom und Jod ist.

Für die Messung unterschiedlicher Ionen kann jeweils ein entsprechend selektiver Ionophor verwendet werden, wobei die funktionellen Gruppen beibehalten werden. Dadurch ist es möglich, für die Messung unterschiedlicher Ionen dieselben spektroskopischen Parameter auszunützen und dieselben Anregungs- und Meßeinrichtungen zu verwenden.

Mit den genannten Ausführungsvarianten lassen sich z.B. folgende spektrospkopische Konzepte verwirklichen.
a) Eine funktionelle Gruppe weist einen Fluorophor und die andere ein aromatisches Molekül auf. Der daraus gebildete Charge-Transfer Komplex fluoresziert nicht. Durch die Aufnahme des Ions durch den Ionophor ändert sich dessen Konformation so, daß der Charge-Transfer Komplex aufgebrochen wird, die Absorptionsbanden des Charge-Transfer Komplexes verschwinden (spektroskopisch zugänglich) und die Fluoreszenz des Fluorophors taucht neu auf. Hier nimmt die Fluoreszenzintensität mit der Konzentration des beobachteten Analyten zu. Das aromatische Molekül wirkt als Fluoreszenzlöscher.
b) Eine funktionelle Gruppe weist einen Fluorophor auf und die andere stellt ein Schweratom dar (z. B. Chlor, Brom oder Jod). Im Ausgangszustand wird die Fluoreszenz des Fluorophors durch den externen Schweratomlöscher gelöscht. Im beladenen Zustand (nach der koordinativen Bindung des Analyten an den Ionophor), kann das Schweratom die Fluoreszenz des Fluorophors nicht mehr löschen. Die Fluoreszenzintensität des Fluorophors wird beobachtet und analytisch ausgewertet.
c) Ausbildung eines klassischen Charge-Transfer-Komplexes bestehend aus einer funktionellen Gruppe mit Elektronendonator (-akzeptor) und einer funktionellen Gruppe mit Elektronenakzeptor (-donator) . Der Charge-Transfer Komplex fluoresziert nicht und zeigt langwellige Absorption. Bei Aufnahme eines Ions durch den Ionophor wird der Extinktionskoeffizient des Komplexes reduziert und es treten neue Absorptionsbanden der einzelnen funktionellen Gruppen hinzu.

Die im Anspruch 1 angeführten Ionophore können für die angegebenen Ionen unter den folgenden Produktnummern bei der Firma FLUKA CHEMIE AG, CH-9470 BUCHS, Schweiz, bezogen werden:

| ION | IONOPHOR | | PRODUKTNUMMER |
|---|---|---|---|
| Li⁺ | Lithium Ionophor | I | 62 557 |
| Na⁺ | Natrium Ionophor | III | 71 734 |
| Ca⁺⁺ | Calcium Ionophor | I | 21 192 |
| Mg⁺⁺ | Magnesium Ionophor | IV | 63 088 |

Erfindungsgemäß werden besonders gute Ergebnisse erzielt, wenn der Abstand der beiden funktionellen Gruppen im Grundskelett des Ionophors mindestens vier, jedoch maximal acht Zwischenatome beträgt.

Ohne Einschränkung der Erfindung werden im folgenden Beispiele für Indikatormoleküle, zur Bestimmung von Li⁺, Na⁺, Ca⁺⁺ und Mg⁺⁺, näher dargelegt.

Bei einem Indikatormolekül zur Bestimmung der Li⁺-Aktivität sind die beiden funktionellen Gruppen an den Positionen R₁ bzw. R₂ mit dem Lithium-Ionophor verbunden. Es handelt sich dabei um ein Phenylendiamin bzw. ein Nitronaphthacen.

Als konkretes Beispiel ist die Substitution mit p-Phenylendiamin bzw. Nitronaphthacen dargestellt, wobei bei Komplexbildung eine neue CT-Bande bei ca. 360 nm auftritt.

### Beispiel 1:

Indikatormolekül mit Lithium-Ionophor I und Nitronaphthacen und Phenylendiamin 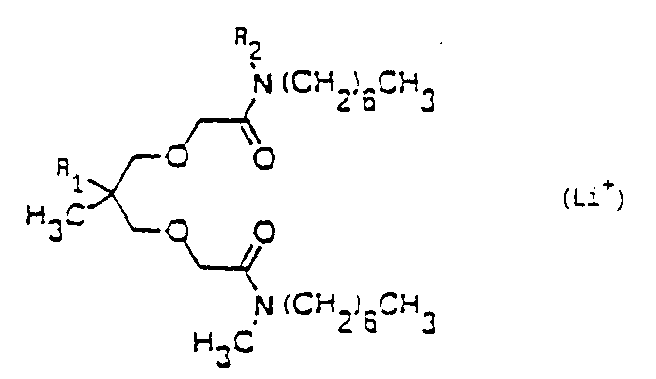 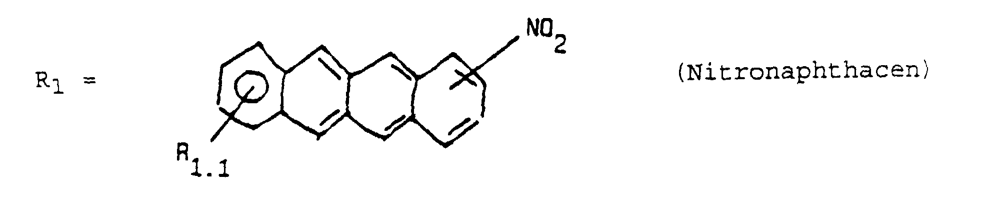 R_{1.1} = (CH₂)ₘ₁- oder - NR_{1.2} - (CH₂)ₙ₁ -
R_{1.2} = -H; - CH₃; - C₂H₅;- C₃H₇
m1 = 0, 1, 2, 3, 4
n1 = 0, 1, 2, 3 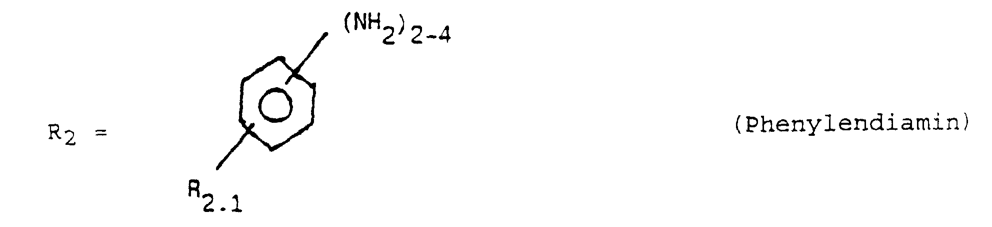 R_{2.1} = -(CH₂)ₘ₂ oder -NR_{2.2} - (CH₂)ₙ₂ -
m2 = 0, 1, 2
n2 = 0, 1
R_{2.2} = -H; - CH₃; C₂H₅; - C₃H₇

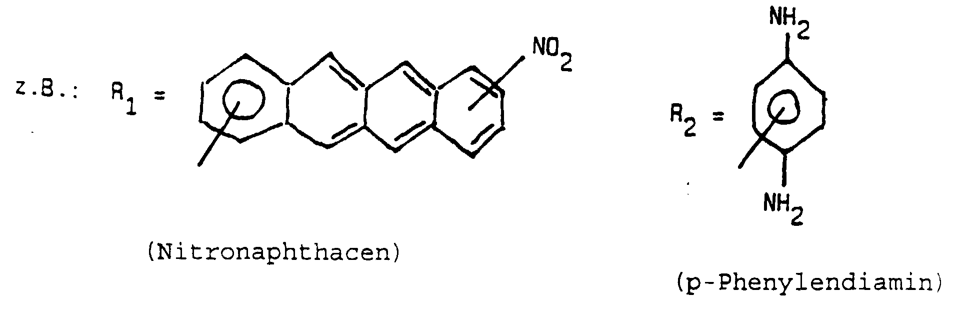

Im folgenden werden Indikatormoleküle für Na⁺ (Beispiel 2 bis 5), Ca⁺⁺ (Beispiel 6) und Mg⁺⁺ (Beispiel 7) angegeben, wobei zu beachten ist, daß die Substitutionsstellen der funktionellen Gruppen (ausgenommen Beispiel 2) auch ausgetauscht werden können. Anstelle der Naphthacene können auch Naphthaline und deren Derivate verwendet werden.

### Beispiel 2:

Indikatormolekuül mit Natrium-Ionophor III und Nitronaphthacen und Phenylendiamin (Variante I)

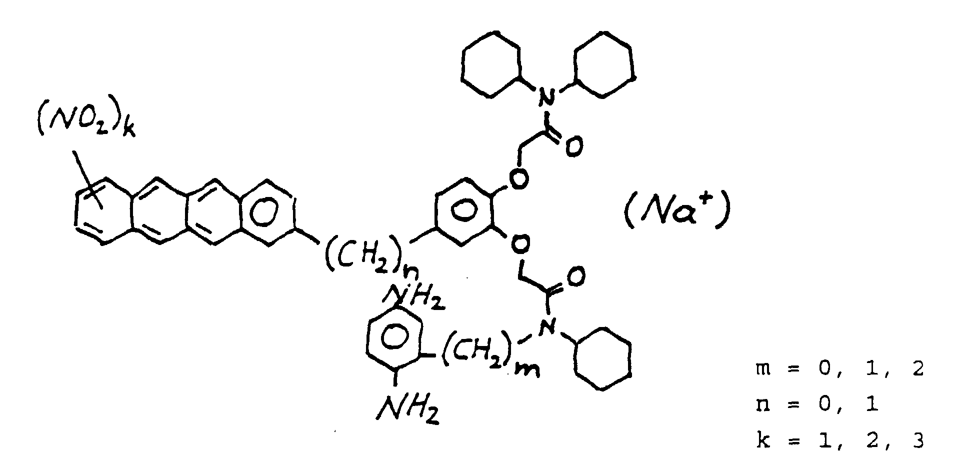

### Beispiel 3:

Indikatormolekül mit Natrium-Ionophor III und Nitronaphthacen und Phenylendiamin (Variante II)

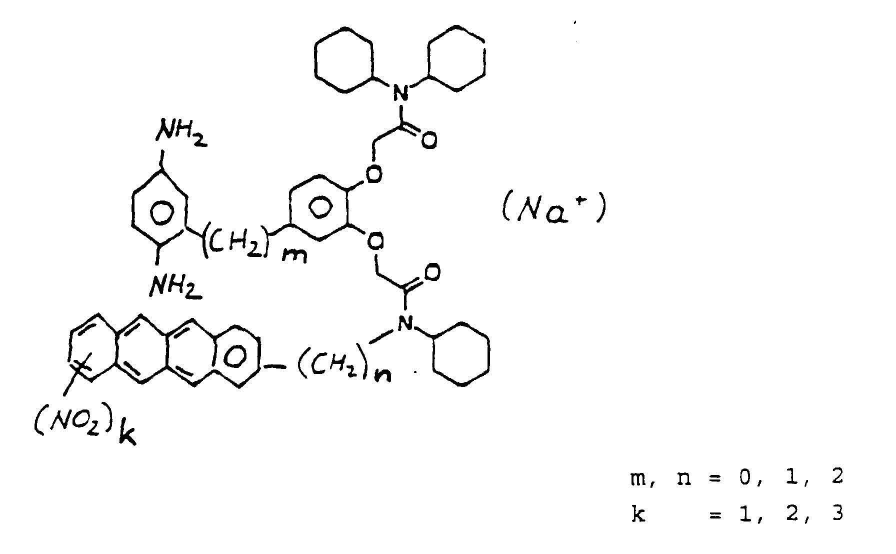

### Beispiel 4:

Indikatormolekül mit Natrium-Ionophor III Hydrochinon als Donor und p-Chinon als Akzeptor

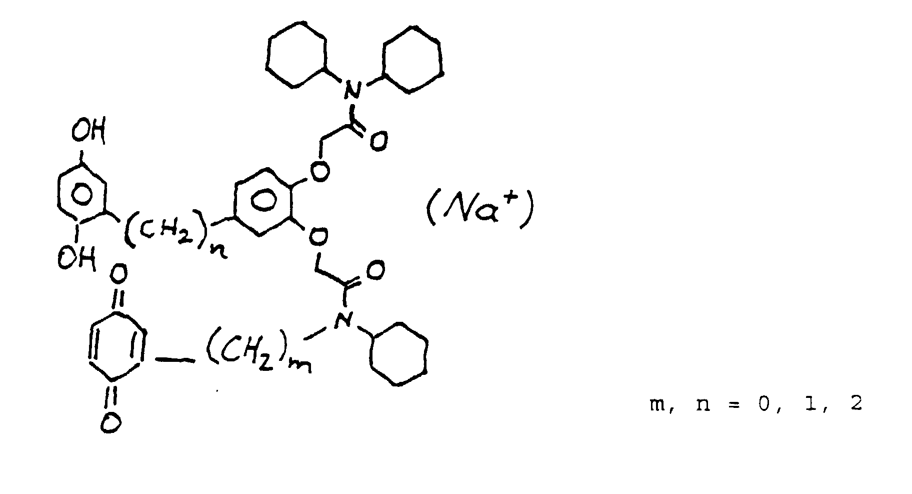

### Beispiel 5:

Indikatormolekül mit Natrium-Ionophor III mit Pyren und dem Schweratom Brom

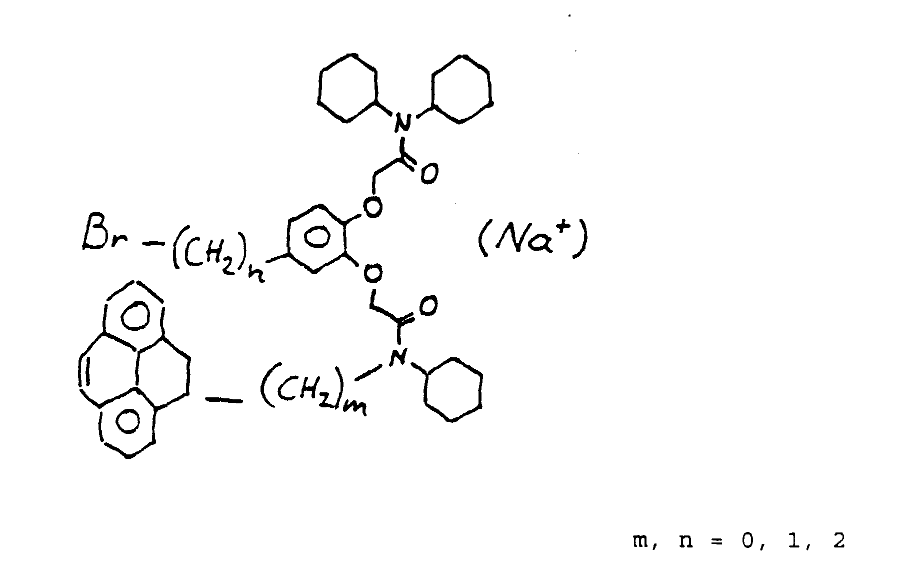

### Beispiel 6:

Indikatormolekül mit Calcium-Ionophor und Nitronaphthacen und Phenylendiamin

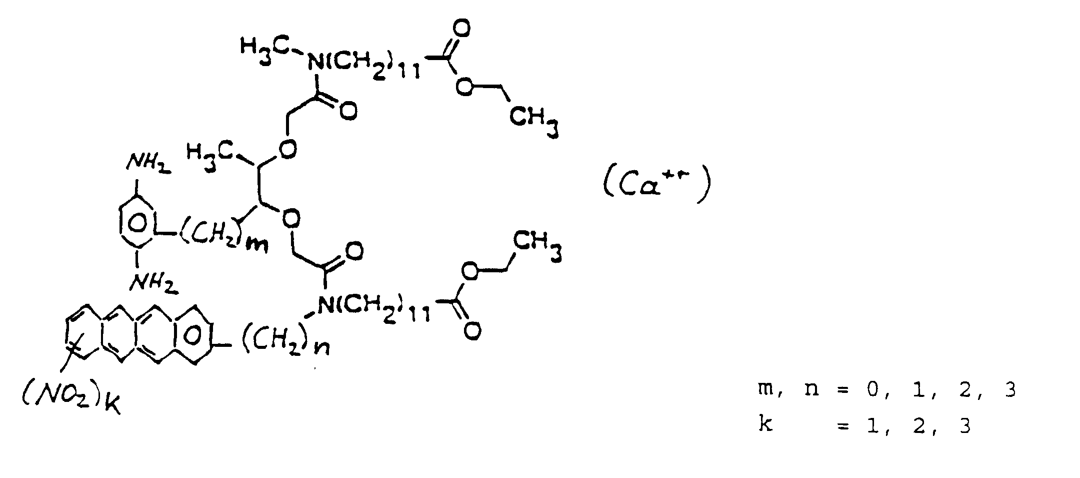

### Beispiel 7:

Indikatormolekül mit Magnesium-Ionophor IV und Nitronaphthacen und Phenylendiamin

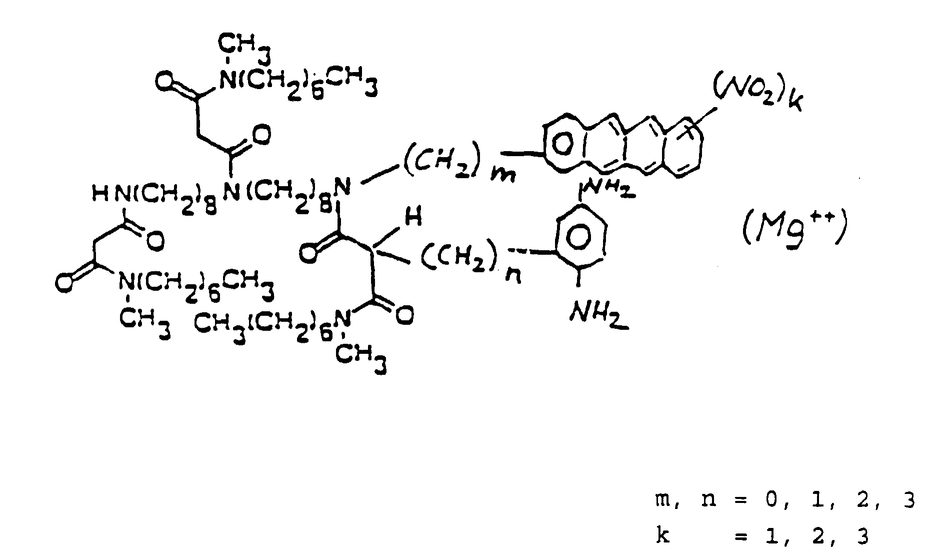

Als Elektronen-Akzeptor können auch mono- bis tri-Nitrocoronen, mono- bis tri-Nitronaphthalin oder mono- bis tri-Nitrobenzo[ghi]perylen in Frage kommen; als Elektronen-Donator mono- bis tri-Aminobenzol, mono- bis tri-Diamethylaminobenzol oder mono- bis tri-Methoxybenzol.

## Patentansprüche

1. Optischer Indikator zur Bestimmung der Aktivität eines Ions in einer Probe, welche den Indikator zumindest indirekt kontaktiert, wobei der Indikator einen Ionophor und zumindest zwei funktionelle Gruppen aufweist, die einen Elektronen-Donator-Akzeptor (EDA) Komplex bilden, dessen Konformation sich durch die Bindung des zu messenden Ions an den Ionophor unter Auflösung des EDA-Komplexes derart ändert, daß sich zumindest eine der optischen Eigenschaften des Indikators meßbar ändert, **dadurch gekennzeichnet**, daß die funktionellen Gruppen an den Ionophor gebunden sind und folgende Ionophore zur Bestimmung der Aktivität folgender Ionen Verwendung finden:
| ION | IONOPHOR |
|---|---|
| Li⁺ | N,N'-Diheptyl-N,N',5,5-tetramethyl-3,7-dioxanonandiamid |
| Na⁺ | N,N,N',N'-Tetracyclohexyl-1,2-phenylendioxydiacetamid |
| Ca⁺⁺ | (-)-(R,R)-N,N'-bis-(11-(ethoxycarbonyl)-undecyl)-N,N'-4,5-tetramethyl-3,6-dioxaoctan-diamid |
| Mg⁺⁺ | N,N',N"-tris-(3-(heptylmethylamino)-3-oxopropionyl)-8,8'-iminodioctylamin |

2. Optischer Indikator nach Anspruch 1, **dadurch gekennzeichnet**, daß beide funktionellen Gruppen des Ionophors aus einem aromatischen Kohlenwasserstoff mit jeweils zumindest einem Substituenten bestehen, wobei der zumindest eine Substituent einer funktionellen Gruppe ein Elektronen-Donator und der zumindest eine Substituent der anderen funktionellen Gruppe ein Elektronen-Akzeptor ist.

3. Optischer Indikator nach Anspruch 1, **dadurch gekennzeichnet**, daß eine funktionelle Gruppe des Ionophors einen aromatischen Kohlenwasserstoff aufweist, dessen zumindest ein Substituent ein Elektronen-Donator ist, sowie daß die andere funktionelle Gruppe des Ionophors ein Atom aus der Gruppe Chlor, Brom und Jod ist.

4. Optischer Indikator nach einem der Ansprüche 2 bis 3, **dadurch gekennzeichnet**, daß zumindest eine funktionelle Gruppe des Ionophors einen Fluorophor aufweist.

5. Optischer Indikator nach einem der Ansprüche 1 bis 4, **dadurch gekennzeichnet**, daß der Abstand der beiden funktionellen Gruppen im Grundskelett des Ionophors mindestens vier, jedoch maximal acht Zwischenatome beträgt.

## Claims

1. Optical indicator for determination of the activity of an ion in a sample, the sample being at least in indirect contact with the indicator, and the indicator having an ionophore and at least two functional groups forming an electron-donor-acceptor (EDA) complex, whose conformation will change as the ion to be measured is bound by the ionophore and the EDA complex is broken up during this process, sucn that at least one of the optical properties of the indicator will undergo a measurable change, **characterized in that** the functional groups are attached to the ionophore, and the following ionophores are used to determine the activity of the following ions:
| ION | IONOPHORE |
|---|---|
| Li⁺ | N,N'-diheptyl-N,N',5,5-tetramethyl-3,7-dioxanonandiamide |
| Na⁺ | N,N,N',N'-tetracyclohexyl-1,2-phenylene dioxydiacetamide |
| CA⁺⁺ | (-)-(R,R)-N,N'-bis-(11-(ethoxycarbonyl)-undecyl)-N,N'-4,5-tetramethyl-3,6-dioxaoctane diamide |
| Mg⁺⁺ | N,N',N"-tris-(3-(heptylmethylamino)-3-oxopropionyl)-8,8-iminodioctylamine |

2. Optical indicator as claimed in Claim 1, **characterized in that** both functional groups of the ionophore consist of an aromatic hydrocarbon with at least one substituent each, the at least one substituent of one functional group being an electron donor and the at least one substituent of the other functional group being an electron acceptor.

3. Optical indicator as claimed in Claim 1, **characterized in that** one functional group of the ionophore has an aromatic hydrocarbon, whose at least one substituent is an electron donor, and that the other functional group of the ionophore is an atom from the group of chlorine, bromine, iodine.

4. Optical indicator as claimed in any of Claims 2 to 3, **characterized in that** at least one functional group of the ionophore has a fluorophore.

5. Optical indicator as claimed in any of Claims 1 to 4, **characterized in that** the distance of the two functional groups in the backbone of the ionophore is at least four and at most eight intermediate atoms.

## Revendications

1. Indicateur optique pour mesurer l'activité d'un ion d'un échantillon, l'ion venant au moins indirectement en contact avec l'indicateur, l'indicateur comprenant un ionophore et au moins deux groupes fonctionnels constituant un complexe donneur - accepteur d'électrons (complexe DAE) dont la conformation se modifie par la fixation de l'ion à mesurer à l'ionophore, accompagnée de la dissociation du complexe DAE, avec comme résultat qu'au moins une des propriétés optiques de l'indicateur est modifiée de manière mesurable, **caractérisé en ce que** les groupes fonctionnels sont fixés sur l'ionophore et en ce que les ionophores suivants sont utilisés pour mesurer l'activité des ions suivants :
| ION | IONOPHORE |
|---|---|
| Li⁺⁺ | N,N'-diheptyl-N,N',5,5-tétraméthyl-3,7-dioxanonane-diamide |
| Na⁺ | N,N,N',N'-tétracyclohexyl-1,2-phénylènedioxydiacétamide |
| Ca⁺⁺ | (-)-(R,R)-N,N'-bis-(11-(éthoxycarbonyl)-undécyl)-N,N'-4,5-tétraméthyl-3,6-dioxaoctane-diamide |
| Mg⁺⁺ | N,N',N"-tris-(3-(heptylméthylamino)-3-oxopropionyl) -8,8'-iminodioctylamine |

2. Indicateur optique selon la revendication 1, **caractérisé en ce que** les deux groupes fonctionnels de l'ionophore sont constitués par un hydrocarbure aromatique, chacun comprenant au moins un substituant, le ou les substituants d'un groupe fonctionnel étant des donneurs d'électrons et le ou les substituants de l'autre groupe fonctionnel étant des accepteurs d'électrons.

3. Indicateur optique selon la revendication 1, **caractérisé en ce qu'**un groupe fonctionnel de l'ionophore présente un hydrocarbure aromatique dont le ou les substituants sont des donneurs d'électrons, et en ce que l'autre groupe fonctionnel de l'ionophore est un atome choisi dans un groupe constitué par le chlore, le brome et l'iode.

4. Indicateur optique selon les revendications 2 à 3, **caractérisé en ce qu'**au moins un groupe fonctionnel de l'ionophore comprend un fluorophore.

5. Indicateur optique selon l'une des revendications 1 à 4, **caractérisé en ce que** la distance entre les deux groupes fonctionnels dans l'ossature de l'ionophore comprend au moins quatre, mais au maximum huit atomes intermédiaires.
